# EUROPÄISCHE PATENTANMELDUNG

(11) **EP 1 371 340 A1**
(43) Veröffentlichungstag der Anmeldung: **17.12.2003**
(21) Anmeldenummer: 03090311.6
(22) Anmeldetag: 13.12.2000
(51) Int. Cl.: A61B 19/00

(54) **System zur Instrumenten- und Knochensegment- sowie Gewebe- und Organnavigation**

(30) Priorität: 13.12.1999 DE 19960020
(62) Teilanmeldung aus: 00983317.9
(71) Anmelder: LB Medical GmbH, 10719 Berlin (DE)
(72) Erfinder: Lüth, Tim, 12203 Berlin (DE); Marmulla, Rüdiger, 69126 Heidelberg (DE)
(74) Vertreter: Hengelhaupt, Jürgen D., Dipl.-Ing.

(57) **Zusammenfassung**

Die Erfindung beschreibt ein Verfahren zur Instrumenten- und Knochensegment- sowie Gewebe- und Organnavigation sowie zur optischen Refenzierung.

Die verallgemeinerte Problemstellung betrifft die Referenzierung zwischen einem Datensatz (der das räumliche Modell eines Körpers geometrisch beschreibt) sowie der realen physikalischen Umwelt in der sich derreale Körper befindet. Für die Referenzierung wird dazu ein dreidimensionaler Lagerreferenzkörper am realen Körper verwendet oder angebracht, der aus einem oder mehreren sensorisch räumlich erfassbaren Elementarkörpern (Markern) besteht, die eine feste geometrische Referenz zum Körperschwerpunkt oder anderen Köreperreferenzvolumen selbst definieren. Zur Registrierung erfolgt eine Zuordnung des Lagerreferenzkörpers bzw. seiner Elementarkörper im Datenmodell und in der physikalischen Welt.

## Beschreibung

Die Erfindung betrifft ein Verfahren entsprechend dem Oberbegriff des Anspruchs 1.

Um bei einem chirurgischen Eingriff eine Referenzierung zwischen Patientendatensatz und Operationssitus vorzunehmen, werden üblicherweise entweder anatomische Landmarken oder - vor Erstellung eines Bilddatensatzes - am Patienten aufgebrachte Implantate (Knochen- oder Hautmarker) verwendet, die jeweils zugleich mit einem Eingabegerät an der Workstation sowie mit einem Lokalisationssystem am Patienten bezeichnet werden.

Die verallgemeinerte Problemstellung betrifft die Referenzierung zwischen einem Datensatz (der das räumliche Modell eines Körpers geometrisch beschreibt) sowie der realen physikalischen Umwelt in der sich der reale Körper befindet. Für die Referenzierung wird dazu ein dreidimensionaler Lagereferenzkörper am realen Körper verwendet oder angebracht, der aus einem oder mehreren sensorisch räumlich erfaßbaren Elementarkörpern (Markern) besteht, die eine feste geometrische Referenz zum Körperschwerpunkt oder anderen Körperreferenzvolumen selbst definieren. Zur Registrierung erfolgt eine Zuordnung des Lagereferenzkörpers bzw. seiner Elementarkörper im Datenmodell und in der physikalischen Welt.

Abweichend von diesem Verfahren beschreibt das Deutsche Patent DE 19747427 ein Verfahren und eine Vorrichtung, bei der die charakteristische Oberfläche von Knochenstrukturen zur Referenzierung zwischen Datensatz und Operationssitus genutzt wird. Dazu beschreibt DE 197 47 427 eine individuelle Schablone, die 3D-Lokalisationsmarker trägt und auf einem Knochenstück aufgesetzt bzw. festgeschraubt wird.

Nachteil dieser Methode ist, daß die Anfertigung einer individuellen Schablone eine kostenintensive Anfertigung eines CAD-CAM-Modells aus dem Patientendatensatz erforderlich macht. Außerdem ist bei zahlreichen chirurgischen Eingriffen eine breite Freilegung von Knochen zum Aufbringen der individuellen Oberflächenschablone unvermeidlich, wodurch der Eingriff unnötig invasiv wird.

Aufgabe der vorliegenden Erfindung ist es, ein Verfahren zur Instrumenten- und Knochensegment- sowie Gewebe- und Organnavigation zu schaffen, welche ohne Hilfsmittel wie Schablonen arbeiten und mit einfachen Mitteln eine sichere, und reproduzierbare Navigation ermöglichen.

Diese Aufgabe wird mit den Merkmalen des Anspruchs 1 gelöst.

Neben der erheblichen Kostenreduktion kann durch eine optische Erfassung und Referenzierung des OP-Situs über die charakteristische Oberfläche eines Weichteilmantels oder einer Knochenoberfläche der operative Zugangsweg geringer invasiv ausfallen. Außerdem Können die 3D-Lokalisierungsmarker unter Verwendung der vorliegenden Erfindung bei einer Knochensegmentnavigation unabhängig von einer Schablone - beispielsweise einer Schraube-einzeln an einem Knochensegment befestigt werden, wodurch sich nochmals Möglichkeiten ergeben, einen operativen Eingriff minimal-invasiv zu gestalten.

Eine optische Referenzierung zwischen Datensatz, Operationssitus und 3D-Lokalisationsmarkern erfolgt zudem rascher und exakter als die eingangs erwähnte Referenzierung über anatomische Landmarken und Implantate, weil sich große Oberflächenstrukturen in einem Patientendatensatz (beispielsweise MRT oder CT) insgesamt exakter abbilden und wiedergeben lassen als kleine, einzelne Referenzpunkte.

Ausführungsbeispiele der Erfindung sind in den Zeichnungen dargestellt und werden im folgenden näher beschrieben.

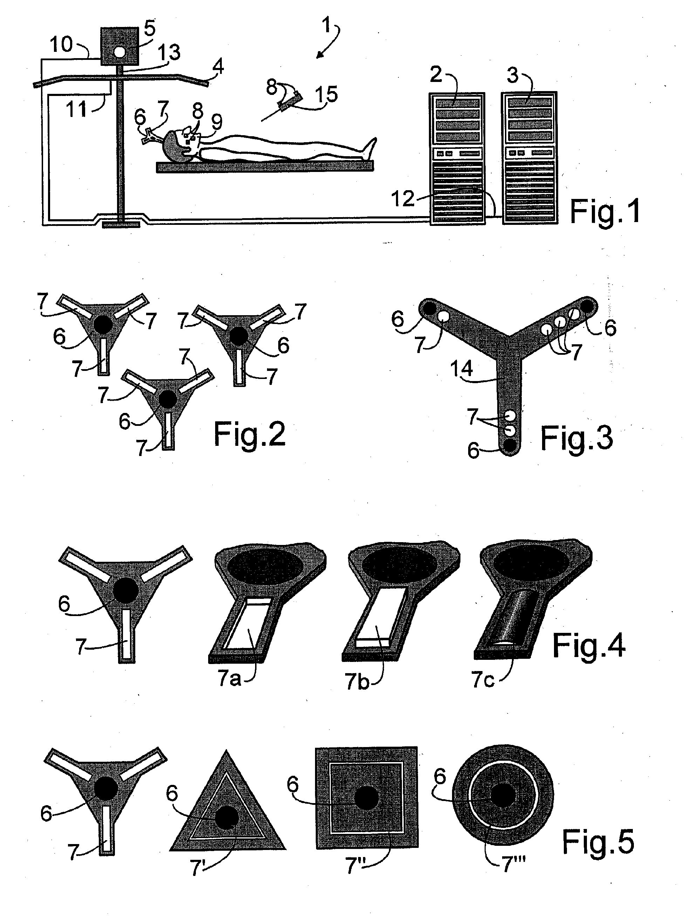

Es zeigen
- Figur 1: eine Ansicht der Vorrichtungen im Operationssaal,
- Figur 2: eine Detailansicht der 3D-Referenzmarker mit Vorrichtung zur Erfassung derselben durch optische Verfahren mittels 3D-Scanner,
- Figur 3: geometrische Vorrichtung zur optischen Erfassung von 3D-Referenzmarkern, die auf einem Rahmen fest aufgebracht sind,
- Figur 4: Perspektivische Darstellung der 3D-Referenzmarker mit zugeordneten geometrischen Vorrichtungen unterschiedlicher Form (Vertiefung/Sulcus, Leiste/Crista, plane farbcodierte Fläche) zur optischen Erfassung mit dem 3D-Scanner,
- Figur 5: alternative, beispielhaft unterschiedliche geometrische Formen mit räumlich bekannter Zuordnung zum 3D-Marker,
- Fig. 6: weitere gekoppelte Referenzierungsmarker,
- Fig. 7: Ausführungsform mit anderer Kopplung zwischen Scanner und Positionserfassungseinheit,
- Fig. 8: Referenzierung nicht notwendigerweise formstabiler Körper,
- Fig. 9: Vorrichtung zur Fixierung nicht notwendigerweise formstabiler Körper.

Die Erfindung wird nachstehend an Hand eines ersten Ausführungsbeispieles näher beschrieben.

Die Gesamtvorrichtung 1 dient der optischen Referenzierung zwischen Operationssitus, Patientendatensatz und 3D-Markern.

An einer Positionserfassungseinheit 4 ist über eine Koppel-Verbindung 13 ein optischer 3D-Scanner 5 befestigt. Die Positionserfassungseinheit 4 kann beispielsweise Infrarotsignale, Ultraschallsignale oder elektromagnetische Signale aufzeichnen und erlaubt die Bestimmung dreidimensionaler Koordinaten eines entsprechenden 3D-Markers 6 (beispielsweise: Ultraschallsender, Infrarotsender, elektromagnetischer Sender bzw. Reflektoren 17 für Wellen aller Art, Ultraschall, Infrarot, Radar etc.). Der 3D-Scanner 5 (beispielsweise ein 3D-Laser-Scanner 5 oder Radar 5a) kann die Form und Farbe von Oberflächen (beispielsweise 7 erfassen, nicht jedoch die Signale der 3D-Marker 6. Die Signale der 3D-Marker 6 können aktiv z.B. durch LED, oder passiv, z.B. durch Reflektoren, ausgesendet werden.

Die von der Positionserfassungseinheit 4 und dem 3D-Scanner 5 oder Radar 5a gemessenen Daten werden über eine Verbindung 10 und 11 an eine Anzeige- und Verarbeitungseinheit 2 weitergeleitet. Weil die Positionserfassungseinheit 4 und der 3D-Scanner 5 über eine Verbindung 13 bekannte geometrische Relationen gekoppelt bzw. kinematisch über eine Verbindung 13 aneinander befestigt sind, lassen sich auf der Verarbeitungseinheit 2 alle mit der Positionserfassungseinheit 4 ermittelten Koordinaten auch im Koordinatensystem des 3D-Scanners 5 und umgekehrt ausdrücken.

Mit der Anzeige- und Verarbeitungseinheit 2 ist über 12 eine Planungseinheit 3 verbunden. Auf dieser Planungseinheit 3 können Operationen simuliert, beispielsweise auch Umstellungsosteotomien vor einer Knochensegmentnavigation geplant werden.

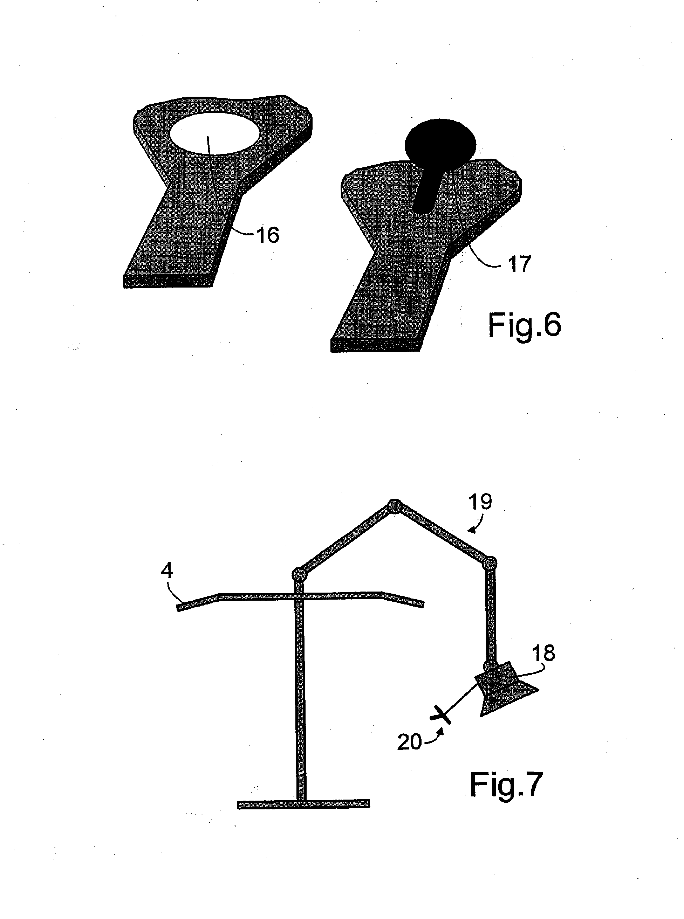

Am Patienten bei diesem Ausführungsbeispiel sind mindestens drei 3D-Marker 6 befestigt, die ein Koordinatensystem am Patienten definieren. In einer bekannten, fixen räumlichen Beziehung zu diesen 3D-Markern 6 sind geometrische Figuren 7 angebracht, die vom 3D-Scanner 5 erkannt werden können. Diese Figuren 7 können beispielsweise als eine Vertiefung/Sulcus 7a, eine erhabene Leiste/Crista 7b, als farbcodierte Linien und Felder 7c oder Strichcode bzw. Barcode ausgebildet sein. Die geometrische Figur 7 kann ebenso auch durch den Sockel, auf dem ein 3D-Marker 6 aufsitzt, gebildet werden. Die geometrische Figur 7 kann ebenso auch direkt durch einen oder mehrere 3D-Marker 6 gebildet werden.

Die Geometrie der Vorrichtungen 7 erlaubt eine eindeutige Rückrechnung der Koordinaten der 3D-Marker 6 auf der Verarbeitungseinheit 2. Die Geometrie dieser Vorrichtungen 7 kann unterschiedlich ausgebildet sein (7', 7'', 7'''), sie muß lediglich vom 3D-Scanner 5 erfaßt und von der Verarbeitungseinheit 2 zur Bestimmung der Koordinaten der 3D-Marker 6 verwendet werden können.

Sind die drei 3D-Marker 6 zur Definition eines Patientenkoordinatensystems über einen Rahmen 14 fest miteinander verbunden, dann können aus der Anordnung geometrischer Figuren 7 auf diesem Rahmen 14 die Koordinaten der 3D-Marker 6 auf der Verarbeitungseinheit 2 errechnet werden.

Der Scanner kann alternativ auch die Analyse der bekannten Geometrien der 3D-Marker direkt dazu verwenden, eine Rückrechnung der Koordinaten zu ermöglichen.

Die Referenzierung zwischen Operationssitus, Patienten-Datensatz und 3D-Markern 6 erfolgt, indem zunächst die Weichteile (vor der Operation, d.h. vor einer Weichteilschwellung oder -verlagerung) oder Knochenoberflächen 9 des Patienten mit dem 3D-Scanner 5 erfaßt werden. Auf der Verarbeitungseinheit 2 werden die Daten des 3D-Scanners 5 verrechnet und die günstigste Oberflächenpaßform zwischen Patient und Patientendatensatz bestimmt. Danach läßt sich mittels Koordinatentransformation eine Referenzierung zwischen Patient und Patientendatensatz herstellen.
Damit sind allerdings noch nicht die 3D-Marker 6 vom 3D-Scanner 5 erfaßt. Da jedoch zusammen mit dem Patient auch die geometrischen Vorrichtungen 7 um die 3D-Marker 6 mitgescannt wurden und weil die räumliche Beziehung zwischen 3D-Markern 6 und geometrischen Vorrichtungen 7 bekannt sind, lassen sich die Koordinaten der 3D-Marker 6 sowohl im Koordinatensystem der vom 3D-Scanner 5 gelieferten Daten als auch im Koordinatensystems des Patientendatensatzes abbilden.

Weitere 3D-Marker 8, die entweder direkt auf einem Knochensegment 9 oder auf einem Arbeitswerkzeug 15 aufgebracht sind oder über eine Meßkinematik oder eine Koordinatenmeßeinrichtung mit diesen gekoppelt sind, lassen sich anschließend im Patientendatensatz auf der Anzeige- und Verrechnungseinheit 2 abbilden.

Damit kann auch ein räumlicher Versatz eines Knochensegments 9, der auf der Planungseinheit 3 simuliert wurde, am Patienten reproduziert werden.

Anstelle einer Kopplung in Form einer festen Verbindung zwischen 3-D-Scanner 5 und 3D-Marker-Positionserfassungseinheit 4 ist es auch möglich, daß der 3D-Scanner 5 mit der Positionserfassungseinheit 4 als Kopplung nicht starr fixiert ist, sondern gegenüber der 3D-Marker-Positionserfassungseinheit 4 mobil bleibt und selbst mit 3D-Markern 8 ausgestattet ist, um von der 3D-Marker-Positionserfassungseinheit 4 registriert werden zu können.

Fig. 6 zeigt zeigt einen 3D-Marker 16 in einer Ausführung als LED und in der Ausführung als passive Reflektorkörper 17. Die 3D-Geometrie der Körper ist soweit bekannt, daß sie zur eindeutigen Rückrechnung der Koordinaten des Markers aus den Scanner-Daten direkt verwendet kann, ohne daß ein zusätzliche Kodierung eingebracht werden muß. Die Marker sind direkt als Vorrichtungsgeometrien geeignet.

Fig. 7 zeigt die Ausführung eines Scanners 18 mit einer als Meßform 19 ausgebildeten Koordinaten-Meßkinematik, die mit der Positionserfassungseinheit direkt verbunden ist. Über die zweite Koordinaten-Meßkinematik kann die relativ Position des Scanners 18 gegebenfalls mit erheblich höherer Genauigkeit und Meßfrequenz erfaßt werden. Alternativ dazu ist die Basis der Koordinaten-Meßkinematik selbst mit einem Lagereferenzkörper 20 ausgestattet. Im einfachsten Fall ist die Koordinaten-Meßkinematik ein einfacher Körper (z.B. Stange) mit bekannter Geometrie. Die Koordinaten-Meßkinematik kann vorteilhaft auch am Tisch oder direkt am Patienten angebracht werden, je nachdem welche Relativgenauigkeit zwischen Markern und Körper maximiert werden soll.

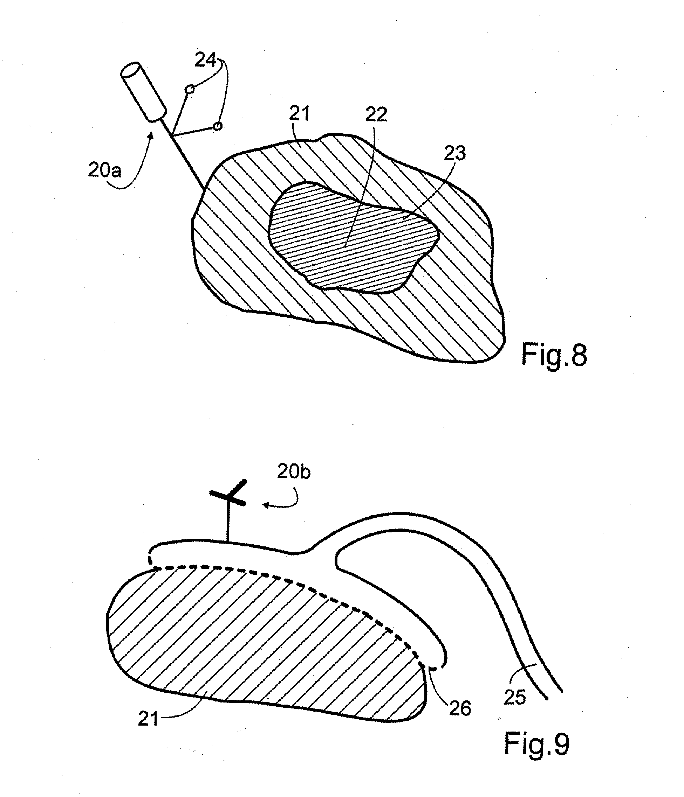

Fig. 8 zeigt anstatt eines Knochens (Hartgewebe) die verallgemeinerte Situation mit nicht notwendigerweise formstabilem Gewebe bzw. einem beliebigen Körper 21. Hier wird im einfachsten Fall eine Relation über einen Körperschwerpunkt 22 oder ein anderes Referenzvolumen 23hergestellt. Dies ist von Vorteil, wenn das Verfahren auch auf Weichgewebe, Organe oder Implantate bei Ausrichtung, Transplantation und Implantation angewendet werden soll. Auch wenn keine perfekte Formstabilität erreicht wird, kann eine Navigationshilfe stattfinden. Am Lagerreferenzkörper 20a sind Elementarkörper 24 angeordnet.

Fig. 9 zeigt eine Vorrichtung zum Fixieren der Lagereferenzkörper 20b an nicht notwendigerweise formstabilen Körpern 21. Der Lagereferenzkörper 20b ist dabei an einem Mechanismus zum befestigen nicht formstabiler Körper 21 angebracht. Im Beispiel wird über ein Unterdruckverfahren durch ein Lumen 25 und durch eine Membran 26 Körpergewebe angesaugt und in eine vorgegebene Form gepreßt. Diese Form kann wiederum vorteilhaft gestaltet werden, um beispielsweise bei der Transplantation oder Implantation das Einfügen zu erleichtern. Andere Verfahren zur Fixierung des Gewebes an der Vorrichtung wie z.B. Kleben, Kletten oder Nähen sind ebenfalls möglich.

## Patentansprüche

1. Verfahren zur Instrumenten- und Knochensegmentsowie Gewebe- und Organnavigation, wobei Positionsdaten von an Knochen, Gewebe oder Organen angeordneten Lagerreferenzkörpern und Konturund/oder Oberflächendaten von an den Lagerreferenzkörpern angeordneten oder räumlich zugeordneten geometrischen Figuren erfasst und die Daten rechentechnisch verarbeibar sind derart, daß die Positionsdaten und die Kontur- und/oder Oberflächendaten in einem gemeinsamen Koordinatensystem darstellbar sind.

2. Verfahren nach Anspruch 1,
**dadurch gekennzeichnet, daß**
die Lagerreferenzkörper eine feste geometrische Referenz zum Körperschwerpunkt oder anderen Körperreferenzvolumen definieren und zur Registrierung eine Zuordnung der Lagerreferenzkörper oder seiner Elementarkörper im Datenmodell und in der physikalischen Welt erfolgt.

3. Verfahren zur optischen Erfassung und Refernezierung, wobei Positionsdaten von Lagerreferenzkörpern und Kontur- und/oder Oberflächendaten von an den Lagerreferenzkörpern angeordneten oder räumlich zugeordneten geometrischen Figuren erfaßt und die Daten rechentechnisch verarbeitet werden derart, daß die Positionsdaten und die Kontur- und/oder Oberflächendaten in einem gemeinsamen Koordinatensystem darstellbar sind.

4. System zur Instrumenten- und Knochensegment- sowie Gewebe- und Organnavigation, wobei Positionsdaten von an Knochen, Gewebe oder Organen angeordneten Lagerreferenzkörpern und Kontur- und/oder Oberflächendaten von an den Lagerreferenzkörpern angeordneten oder räumlich zugeordneten geometrischen Figuren erfassbar und die Daten rechentechnisch verarbeibar sind derart, daß die Positionsdaten und die Kontur- und/oder Oberflächendaten in einem gemeinsamen Koordinatensystem darstellbar sind.
